# EUROPEAN PATENT APPLICATION

(11) **EP 4 360 641 A1**
(43) Date of publication of application: **01.05.2024**
(21) Application number: 22827666.3
(22) Date of filing: 23.06.2022
(51) Int. Cl.: A61K 38/00, A61P 19/06

(54) **USE OF MAZDUTIDE**

(30) Priority: 25.06.2021 CN 202110711050
(71) Applicant: Innovent Biologics (Suzhou) Co., Ltd., Suzhou, Jiangsu 215123 (CN)
(72) Inventor: AN, Pei, Suzhou, Jiangsu 215123 (CN); DENG, Huan, Suzhou, Jiangsu 215123 (CN)
(74) Representative: Nederlandsch Octrooibureau
(86) International application number: PCT/CN2022/100878
(87) International publication number: WO 2022/268174

(57) **Abstract**

The present invention discloses use of mazdutide, more particularly, use of a compound of formula (I) in preparing a medicament for reducing a uric acid level in a patient. Formula (I) is shown in the specification. Mazdutide of the present invention has a significant effect of reducing uric acid.

## Description

### PRIORITY AND RELATED APPLICATION

The present application claims priority to Chinese Patent Application No. 202110711050.0 entitled "USE OF OXM3" filed on June 25, 2021, which is incorporated herein by reference in its entirety along with the appendix.

### TECHNICAL FIELD

The present invention relates to the field of pharmaceuticals, specifically, to the field of uric acid reduction, and more specifically, to use of mazdutide.

### BACKGROUND

An elevated uric acid level is a metabolic disease caused by elevated blood uric acid levels due to dysfunction of purine metabolism. Generally, the daily production amount and excretion amount of uric acid in human bodies are approximately equal. For production, one-third of uric acid is from food, and two-thirds of uric acid is synthesized by human bodies. As for excretion, one-third of uric acid is excreted via intestinal tracts, and two-thirds of uric acid is excreted via kidneys. If any of the above routes fails, the uric acid level will be elevated.

Elevated uric acid levels are generally associated with obesity and diabetes. Obesity is a state of metabolic dysfunction, and is associated with hyperinsulinemia and insulin resistance, leading to elevated levels of circulating adipocyte factors; type 2 diabetes is a disease mainly caused by the dysfunction of glucose metabolism, and is characterized by chronic blood glucose increase caused by insulin resistance and progressive pancreatic β cell dysfunction. The two diseases are involved in insulin metabolism, whereas the metabolic effect of insulin on glucose and fat is influenced by a plurality of adipocytes, which further enhance insulin resistance. Eventually, the production of uric acid and the reabsorption of uric acid by renal tubules are increased, resulting in increased uric acid levels. In addition to obesity and diabetes, elevated uric acid levels may cause other complications such as uremia, atherosclerosis, hypertension, and the like. Elevated uric acid levels may also lead to other diseases, such as hyperuricemia and gout. Hyperuricemia (defined as 2 blood uric acid measurements over 420 µmol/L on different days, regardless of males or females, according to Chinese Diagnosis and Treatment Guidelines for Hyperuricemia and Gout (2019)) and gout are independent risk factors of diseases such as chronic kidney disease, hypertension, cardiovascular and cerebrovascular diseases and diabetes, and are independent predictors of premature death (refer to Bardin T, Richette P., Impact of Comorbidities on Gout and Hyperuricemia: an Update on Prevalence and Treatment Options [J]. BMC Med.,2017,15(1):123).

The GLP-1R/GCGR dual agonist
glucagon-like peptide-1 (GLP-1) is a peptide hormone secreted in intestinal tracts and has a variety of mechanisms for reducing blood glucose and weight, including the effects of increasing glucose-dependent insulin secretion, inhibiting glucagon secretion, delaying gastric emptying, and suppressing central appetite.

Glucagon is a hormone secreted by islet α cells and consists of a single-chain polypeptide of 29 amino acids in length. Glucagon exerts its physiological effects by specifically binding to glucagon receptor (GCGR) on the surface of target cells in the liver and kidneys, activating adenylate cyclase in the cells, and increasing the intracellular cAMP level. Glucagon is a hormone promoting catabolism, and the short-term administration of glucagon can promote glycogenolysis and gluconeogenesis, thus increasing blood glucose. However, it was found that the long-term activation of GCGR by glucagon injection may lead to decreased appetite, stimulated fatty acid decomposition, and significantly increased energy expenditure in adipose tissues (see Campbell JE, Drucker DJ., Nature Reviews Endocrinology, 2015, 11(6):329-338).

Endogenous oxyntomodulin (OXM) is a peptide hormone secreted by L cells of the human intestinal tracts after nutritional intake. OXM is a dual agonist of glucagon-like peptide-1 receptor (GLP-1R) and glucagon receptor (GCGR). It combines the anorexic and hypoglycemic effects of GLP-1R agonists with the GCGR-mediated energy expenditure increasing effect (see Pocai A. Unraveling Oxyntomodulin, GLP1's Enigmatic Brother, J Endocrinol., 2012;15:335-346; Day JW, Ottaway N, Patterson JT, et al., A New Glucagon and GLP-1 Co-Agonist Eliminates Obesity in Rodents, Nat Chem Biol., 2009;5:749-757), and may thus be superior to the GLP-1R agonists in treating obesity and reducing blood glucose. OXM injection in humans can significantly reduce body weight and appetite, and increase energy expenditure. GLP-1R-knockout mice (GLP-1R-/-) receiving slow infusions of OXM were found to have milder weight loss compared to the wild-type (WT) mice. This indicates that the weight-lowering effect of OXM requires the activation of both GLPIR and GCGR (Kosinski JR, Huber J, Carrington PE, et al., The Glucagon Receptor is Involved in Mediating the Body Weight-Lowering Effects of Oxyntomodulin, Obesity, 2012;20:1566-1571). Preclinical data in rodents indicate that GLP-1R/GCGR agonists are more effective in reducing body weight than GLP-1R agonists. Likewise, Lao et al. reported that their dual GLP-1R/GCGR agonists showed higher weight-lowering effects in diet-induced obese rhesus monkeys (see Lao J, Hansen BC, DiMarchi R, et al., Effect of GLP1R/GCGR Dual Agonist in Monkeys, Diabetes, 2013;62(suppl 1):A257; Ralf Elvert, Andreas W. Herling, Running on Mixed Fuel-Dual Agonistic Approach of GLP-1 and GCG Receptors Leads to Beneficial Impact on Body Weight and Blood Glucose Control: A Comparative Study Between Mice and Non-Human Primate, Diabetes Obes Metab., 2018;20:1836-1851). In animals receiving OXM treatment, the activation of GCGR in the central nervous system may improve systemic glucose metabolism (see Mighiu PI, Yue JT, Filippi BM & Lam TK, 2012, Hypothalamic Glucagon Signaling Regulates Glucose Production, Diabetes, 61 (Suppl 1) A55; Nauck MA, 2012, The Design of the Liraglutide Clinical Trial Programme, Diabetes, Obesity and Metabolism, 14 (Suppl 2) 4-12). The hypoglycemic effect of OXM may be mainly attributed to the effects of reducing body weight, promoting insulin secretion, and activating the GCGR in the central nervous system to inhibit hepatic glucose production.

These data indicate that OXM has the potential to be a well-tolerated anti-obesity and hypoglycemic agent. However, there is currently no relevant study on the effect of OXM on uric acid levels, though some uric acid lowering agents, such as xanthine oxidase inhibitors (XOIs) and uricosuric agents, are present on the market today. Among these, allopurinol, febuxostat, and benzbromarone are first-line therapies approved in China. In the U.S., only allopurinol has been approved as a first-line treatment. However, existing uric acid-lowering drugs have problems in efficacy or safety, for example, severe hypersensitivity rate of allopurinol, cardiovascular risk of febuxostat, adverse effects of benzbromarone, insufficient efficacy, and the like. Therefore, existing uric acid-lowering drugs for treating gout cannot meet the clinical requirements. As such, a uric acid-lowering formulation with high safety and tolerability and a significant effect of reducing uric acid is urgently needed at present, particularly in reducing uric acid in patients with obesity or diabetes.

### SUMMARY

In order to solve the problems of the absence of uric acid-lowering formulation with high safety and tolerability and a significant effect of reducing uric acid in the prior art, the present invention provides use of mazdutide, which has a significant effect of reducing uric acid.

Mazdutide in this study is an OXM analog. Mazdutide is a synthetic long-acting peptide analog to mammalian oxyntomodulin (OXM) that utilizes a fatty acyl side chain to extend the duration of action, allowing once-weekly dosing. When administered, OXM increases glucose tolerance and leads to weight loss (Pocai A., Action and Therapeutic Potential of Oxyntomodulin, Mol Metab., 2013;3(3):241-251). In humans, this hormone is thought to exert its biological effects by activating glucagon-like peptide-1 receptor (GLP-1R) and the glucagon receptor (GCGR) (see Tan TM, Coadministration of Glucagon-Like Peptide-1 During Glucagon Infusion in Humans Results in Increased Energy Expenditure and Amelioration of Hyperglycemia, Diabetes, 2013;62(4):1131-1138). As an OXM analog, the action of mazdutide is thought to be mediated by the binding and activation of GLP-1R and GCGR.

The present invention provides use of a compound of formula (I) (mazdutide) or a pharmaceutically acceptable salt thereof in preparing a medicament for reducing a uric acid level in a patient;

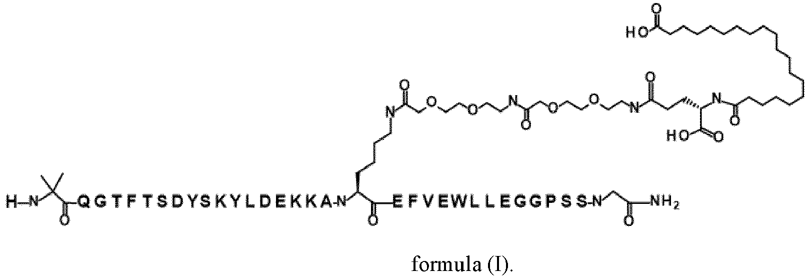

The compound or the pharmaceutically acceptable salt thereof is preferably the sole active ingredient or one of the active ingredients in the medicament.

The medicament preferably further comprises tris(hydroxymethyl)aminomethane and mannitol, and more preferably further comprises sucrose or propylene glycol.

Furthermore, the serum uric acid level in the patient is preferably greater than 280 µmol/L before the administration of the compound.

Alternatively, the patient has gout, hyperuricemia, uremia, atherosclerosis, hypertension, fatty liver, diabetes, obesity, or overweight with complications.

In certain cases, the patient has both a serum uric acid level of greater than 280 µmol/L and the above conditions.

In certain embodiments, the uric acid level is greater than 420 µmol/L.

The present invention further provides a method for reducing a uric acid level in a patient, comprising administering to the patient the compound of formula (I) or the medicament as defined above.

The present invention further provides a method for treating gout, comprising administering to a patient the compound of formula (I) or the medicament as defined above.

With respect to the administered dose of the above medicament, the medicament is administered at a dose of 1.0-10 mg once weekly; preferably, the medicament is administered at a dose of about 1.0 mg, 1.5 mg, 2.0 mg, 2.5 mg, 3.0 mg, 4.0 mg, 4.5 mg, 5 mg, 6 mg, 7.5 mg, 9 mg, or 10 mg once weekly.

In one embodiment, the medicament is administered at at least one ascending dose about once weekly for at least about four weeks, and administered at at least one maintenance dose about once weekly for at least about four weeks after the ascending dose; wherein the ascending dose is selected from about 1.0 mg and about 2.0 mg; wherein the maintenance dose is selected from about 3.0 mg.

In one embodiment, the medicament is administered at at least one ascending dose about once weekly for at least about four weeks, and administered at at least one maintenance dose about once weekly for at least about four weeks after the ascending dose; wherein the ascending dose is selected from about 1.5 mg and about 3.0 mg; wherein the maintenance dose is selected from about 4.5 mg.

In one embodiment, the medicament is administered at at least one ascending dose about once weekly for at least about four weeks, and administered at at least one maintenance dose about once weekly for at least about four weeks after the ascending dose; wherein the ascending dose is selected from about 2.0 mg and about 4.0 mg; wherein the maintenance dose is selected from about 6.0 mg.

In another aspect of the present invention, provided is a pharmaceutical composition for reducing a uric acid level in a patient, comprising a compound of formula (I) or a pharmaceutically acceptable salt thereof, and at least one pharmaceutically acceptable carrier.

Earlier studies show that mazdutide has good safety and tolerability in single-dose and multi-dose escalation studies in healthy populations, and has the effect of reducing uric acid in patients with obesity or diabetes. A single dose study in healthy subjects (I8P-MC-OXAA): a single center, double-blind, randomized, placebo-controlled SAD study has been completed (ascending doses of 0.03 mg, 0.1 mg, 0.3 mg, 1.0 mg, 2.5 mg, and 5.0 mg), which mainly assessed the safety, tolerability, and PK/PD profile of mazdutide in healthy subjects. The results show that: the drug was tolerated in subjects when the dose ascended to 2.5 mg, but gastrointestinal-related AEs (mainly nausea and vomiting) were observed in 6 subjects when the dose ascended to 5 mg; therefore, 2.5 mg was determined as the maximum tolerated dose (MTD) for single-dose administration.

As used herein, the "ascending dose" refers to a dose that is less than the maximum effective dose required in a patient.

As used herein, the "maintenance dose" refers to a dose as the maximum effective dose required in a patient. As used herein, the "pharmaceutically acceptable salt" is well known to those skilled in the art. In one embodiment, the pharmaceutically acceptable salt is a trifluoroacetate.

As used herein, the "patient" refers to a mammal in need of treatment for a condition or disorder. In one embodiment, the patient is a human with a disease or condition that would benefit from mazdutide treatment. As used herein, the term "about", when present in connection with a number, may refer to, for example, ± 5%, ± 4%, ± 3%, ± 2%, ± 1%, or ± 0.5%.

References to amino acids as used herein are well known to those skilled, such as: Ala (A), Val (V), Leu (L), Ile (I), Pro (P), Phe (F), Trp (W), Met (M), Gly (G), Ser (S), Thr (T), Cys (C), Tyr (Y), Asn (N), Gln (Q), Asp (D), Glu (E), Lys (K), Arg (R), and His (H).

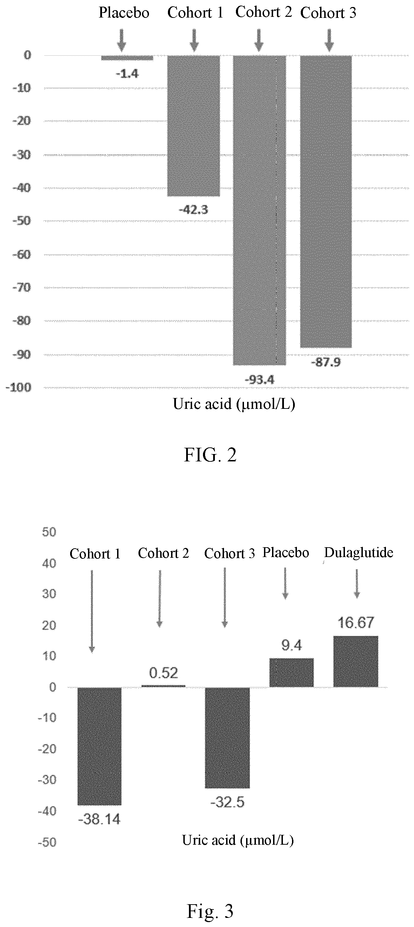

Preliminary study results in patients with overweight or obesity show that: mazdutide can reduce the uric acid level in patients (see FIG. 2 for details). Preliminary study results in patients with type 2 diabetes show that: mazdutide can also decrease the uric acid level in such patients (see Table 6 and FIG. 3 for details). The beneficial effects of the present invention are as follows:
Mazdutide has a significant effect of reducing uric acid, and can reduce the uric acid level in a hyperuricemic patient by more than 80 µmol/L.

### BRIEF DESCRIPTION OF THE DRAWINGS

FIG. 1 illustrates the design of a related study.
FIG. 2 illustrates the change in uric acid from baseline after administration in subjects with overweight/obesity.
FIG. 3 illustrates the change in uric acid from baseline after administration in subjects with diabetes.

### DETAILED DESCRIPTION

The present invention is further illustrated by the following examples, which, however, should not be construed as limiting the present invention. Experimental procedures without specified conditions in the following examples are conducted in accordance with conventional procedures and conditions, or in accordance with the manufacturer's manual.

### Example 1. Study drugs and subjects

The structure of mazdutide is represented by formula (I)

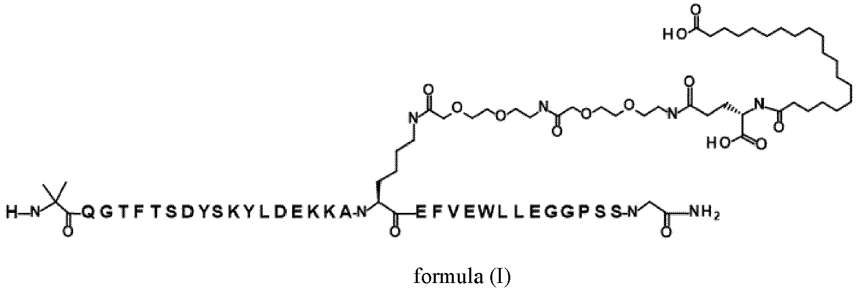

The sequence is set forth in SEQ ID NO: 1, and the specific sequence is as follows: His-Xaa-Gln-Gly-Thr-Phe-Thr-Ser-Asp-Tyr-Ser-Lys-Tyr-Leu-Asp-Glu-Lys-Lys-Ala-Lys-Glu-Phe-Val-Glu-Trp-Leu-Leu-Glu-Gly-Gly-Pro-Ser-Ser-Gly, wherein Xaa is Aib (2-aminoisobutyric acid); the Lys at position 20 is chemically modified by conjugating with ε-amino group of the Lys side chain via ([2-(2-amino-ethoxy)-ethoxy]-acetyl)₂-(γGlu)₁-CO-(CH₂)₁₈-CO₂H, and the carboxyl group of the C-terminal Gly was amidated to a C-terminal primary amide.

### 1.1 The physical and chemical characteristics of the investigational product are shown in Table 1:

**Table 1**

| | |
|---|---|
| Molecular weight: | 4560.32 Dalton |
| Appearance: | White to off-white powder |
| pI: | 5.2 |
| Stability: | The drug substance is stable at ≤ -20 °C. |
| Solubility: | In 20 mM tris(hydroxymethyl)aminomethane solutions and 150 mM NaCl solutions with pH 8.0, the solubility of mazdutide is ≥ 10 mg/mL. |

### 1.2 Specifications and manufacturer of study drugs

The mazdutide preparation is mazdutide for injection consisting of 2 mg of mazdutide and inactive ingredients tris(hydroxymethyl)aminomethane, mannitol, and sucrose. The contents in the vial were reconstituted in sterile water for injection to give a clear solution of mazdutide. Alternatively, the preparation may be formulated with mazdutide and inactive ingredients tris(hydroxymethyl)aminomethane, mannitol and propylene glycol, and the preparation method is described in PCT/CN2022/089742.

The placebo was a mazdutide mimic, consisting of the inactive ingredients tris(hydroxymethyl)aminomethane, mannitol and sucrose. The contents in the vial were reconstituted in sterile water for injection to give a clear solution of the inactive ingredients.

The specification of the formulations was 2 mg/vial, and the placebo was in the same specification as the investigational formulation.

Dulaglutide: 1.5 mg/vial, manufactured by: Vetter Pharma-Fertigung GmbH & Co. KG.

### 1.3 Storage

The formulation and placebo should be stored under refrigerated conditions (2 °C to 8 °C).

### 1.4 Administration

Mazdutide and placebo were both administered subcutaneously once weekly. The doses were prepared and administered by study nurses according to the manual instructions of the study drugs.

### 1.5 Inclusion criteria

I. Subjects with obesity or overweight meeting all of the following criteria were included in the study:
   1. Aged 18-75 years (inclusive), male or female;
   2. For obesity, BMI ≥ 28.0 kg/m²; for overweight, 24 ≤ BMI < 28.0 kg/m² with at least one of the following: i, increased appetite, intolerable hunger before meals, and increased food consumption; ii, comorbidity with one or more of pre-diabetes (impaired fasting glucose and/or impaired glucose tolerance), hypertension, dyslipidemia (see appendix 4 for reference criteria), and fatty liver (within 6 months before the screening); iii, comorbidity with weight-bearing joint pain; iv, obesity-related dyspnea or obstructive sleep apnea syndrome;
   3. Weight change < 5% after at least 12 weeks of diet/exercise weight control at the time of screening Percentage weight change = |(weight at screening - weight at 12 weeks before screening)/weight at seteening| × 100%
   4. Capable of understanding the procedures and methods of the study, and willing to comply with the clinical protocol strictly to complete the study and to provide informed consent.
II. Subjects with diabetes meeting the following criteria were included in the study:
   1. Confirmed type 2 diabetes for at least 6 months according to the WHO criteria 1999.
   2. Male or female aged from 18-75 (inclusive) when providing informed consent.
   3. Patients with uncontrolled type 2 diabetes despite lifestyle intervention or stable doses of metformin (≥1000 mg/day or the maximum tolerated dose) within 2 months before the screening.
   4. An HbA1c of 7.5%-11.0% measured by a local laboratory at the time of screening.
   5. A body mass index (BMI) of 20-35 kg/m² (BMI = weight (kg)/height (m)²).
   6. Capable of maintaining the original diet, exercise, and lifestyle during the study.
   7. Voluntary informed consent and willingness to strictly comply with the protocol.

### 1.6 Exclusion criteria

I. Subjects with obesity or overweight meeting any of the following criteria were excluded from the study:
1. Suspected allergy to the study drugs or components or allergic predisposition;
2. Use of any of the following drugs or treatments before the screening:
1) previous use of either GLP-1 receptor (GLP-1R) agonists or GLP-1R/GCGR agonists;
2) use of drugs that may have an effect on body weight within 3 months before the screening, including: systemic steroid hormone therapy (intravenous, oral, or intraarticular administration), metformin, SGLT2 inhibitors, thiazolidinediones (TZD), tricyclic antidepressants, psychotropic agents, or sedatives (e.g., imipramine, amitriptyline, mirtazapine, paroxetine, phenelzine, chlorpromazine, thioridazine, clozapine, olanzapine, valproic acid, valproic acid derivatives, and lithium salts), and the like;
3) use of Chinese herbal medicines or health care products that may have an effect on body weight within 3 months before the screening.
4) previous or current use of weight-loss drugs within 3 months before the screening, such as: sibutramine hydrochloride, orlistat, phentermine, phenylpropanolamine, mazindol, phentermine, amfepramone, lorcaserin, compounded phentermine/topiramate, compounded naltrexone/amfepramone mixture, etc.;
5) participation in other clinical trials (reception of the study treatment) within 3 months before the screening;

3. A history or evidence of any of the following diseases before the screening:
1) confirmed diabetes according to WHO criteria 1999;
2) fasting venous blood glucose ≥ 7.0 mmol/L at the time of screening or venous blood glucose ≥ 11.1 mmol/L at two hours after the sugar load of a 75 g oral glucose tolerance test (OGTT) (subjects with fasting blood glucose of 6.1-7.0 mmol/L at the time of screening were confirmed by the venous blood glucose at two hours after the sugar load of the OGTT);
3) previous or current retinopathy at the time of screening;
4) secondary diseases or drug-induced obesity, including: cortisol hormone elevation (e.g., Cushing's syndrome), obesity caused by pituitary and hypothalamus injury, obesity caused by reduction/withdrawal of weight-loss drugs, etc.;
5) previous bariatric surgery, or acupuncture for weight loss within 1 year before the screening;
6) a history of depression; or a history of serious psychiatric disorders, for example: schizophrenia, bipolar disorder, and the like;
7) uncontrolled hypertension at the time of screening despite at least 4 weeks of hypotensive treatment, defined as: a systolic blood pressure of > 140 mmHg and/or a diastolic blood pressure of > 100 mmHg;
8) a systolic blood pressure of < 90 mmHg and/or a diastolic blood pressure of < 50 mmHg at the time of screening;
9) a history of malignancy (except for cured basal cell carcinoma and cervical carcinoma *in situ*) at the time of screening;
10) cardiac diseases (such as angina pectoris, myocardial infarction, cardiomyopathy, acute and chronic heart failure, etc.) at the time of screening;
11) hemorrhagic or ischemic stroke or transient ischemic attack within 6 months before the screening;
12) a history or family history of medullary thyroid cancer, or MEN (multiple endocrine neoplasia) 2A or 2B syndrome at the time of screening;
13) a history of acute or chronic pancreatitis, gallbladder disease and pancreas injury at the time of screening;
14) chronic gastrointestinal diseases and systemic diseases that may affect gastrointestinal motility at the time of screening, or use of drugs that may change gastrointestinal motility, appetite or absorption within 3 months before the screening;
15) limb deformity or defects that may affect the accurate measurement of indexes such as height, weight, and the like;
16) ineligibility as determined by the investigator due to major and medium surgery, serious trauma, or serious infection within 1 month before the screening;
17) previous suicidal tendency or suicidal behavior;
18) scheduled surgery during the study, except for outpatient surgeries that do not affect the safety of the subject or the study results as determined by the investigator;
19) human immunodeficiency virus (HIV) antibody, hepatitis B surface antigen (HBsAg), or Hepatitis C virus (HCV) antibody, or syphilis antibody positive at the time of screening;
20) a history of alcohol abuse within 1 month before the screening. Alcohol abuse is defined as: over 21 units for men or over 14 units for women per week on average, or unwillingness to cease drinking within 24 hours before dosing and throughout the study period (1 unit = 360 mL of beer, or 150 mL of wine, or 45 mL of distilled spirits);
21) drug abuse and positivity in a urine test for drugs at the time of screening;

4. Any one of the laboratory tests meets the following criteria (those of proper reasons at the time of screening can be re-examined within one week, and the reason for retesting should be documented by the investigator):
1) serum calcitonin ≥ 15 ng/L at the time of screening;
2) at the time of screening, alanine aminotransferase ≥ 2.0 × ULN and/or aspartate aminotransferase ≥ 2.0 × ULN and/or total bilirubin ≥ 1.0 × ULN and/or alkaline phosphatase ≥ 2.0 × ULN;
3) at the time of screening, eGFR < 60 mL/min/1.73 m², as estimated using the CKD-EPI equation (see Table 2 below): CKD-EPI equation: eGFR = a × [(blood creatinine (µmol/L)/b)] c × (0.993) age:

**Table 2**

| **Gender** | **a value** | **b value** | **c value** | |
|---|---|---|---|---|
| | | | Serum creatinine ≤ 0.7 mg/dL | Serum creatinine ≥ 0.7 mg/dL |
| Female | 144 | 0.7 | -0.329 | -1.209 |
| Male | 141 | 0.9 | -0.411 | -1.209 |

4) abnormal thyroid function (FT3, FT4, or TSH) at the time of screening;
5) fasting triglyceride ≥ 5.64 mmol/L (500 mg/dL) at the time of screening;
6) blood amylase or lipase > 2.0 × ULN at the time of screening;
7) an international normalized ratio (INR) of prothrombin time greater than the upper limit of the normal range at the time of screening;
5. Heart rate < 50 beats/min or > 90 beats/min as measured by 12-lead electrocardiogram (ECG) at the time of screening;
6. Clinically significant abnormality in 12-lead ECG at the time of screening: atrioventricular block degree II or degree III, long QT syndrome or QTcF > 450 ms (QTc Fridericia equation: QTcF = QT/(RR^0.33)), PR interval < 120 ms or PR interval > 220 ms, QRS > 120 ms, left or right bundle branch block, pre-excitation syndrome or severe arrhythmia requiring treatment in the absence of cardiac pacemaker;
7. Pregnant or lactating women, or men or women with fertility unwilling to take contraceptive measures throughout the study period;
8. Blood donation and/or blood loss of ≥ 400 mL or bone marrow donation within 3 months before the screening, or the presence of hemoglobinopathy, hemolytic anemia, sickle-cell anemia, or hemoglobin < 110 g/L (male) or < 100 g/L (female);
9. Ineligibility due to any other factors that might affect the efficacy or safety assessment of the study as determined by the investigator.
II. Subjects with diabetes meeting any of the following criteria were excluded from the study:
1. Type 1 diabetes, specific types of diabetes, or gestational diabetes.
2. Ketoacidosis or lactic acidosis within 6 months before the screening.
3. A history of severe hypoglycemic episodes within 6 months before the screening, defined as the presence of symptoms of neural hypoglycemia and requiring the help of others for recovery, or complete unawareness of hypoglycemia or insufficient awareness of hypoglycemic symptoms. Patients with difficulties in communication or understanding hypoglycemic symptoms and proper treatments as determined by the investigator should also be excluded.
4. Acute myocardial infarction, unstable angina, coronary artery bypass graft, percutaneous coronary intervention (except diagnostic angiography), transient ischemic attack (TIA), cerebrovascular accident, acute or chronic heart failure within 6 months before the screening.
5. Abnormality in 12-lead ECG (e.g., QTcF > 450 ms, PR interval < 120 ms or PR interval > 220 ms, atrioventricular block degree II or degree III, delayed ventricular conduction (QRS > 120 ms), right bundle branch block, left bundle branch block, pre-excitation syndrome) that may increase risks in subjects or affect ECG data analysis (QT) as determined by the investigator at the time of screening; or current use of any drug that may affect the QT interval (e.g., class IA and III antiarrhythmic agents, cisapride, macrolide antibiotics, and psychotropic agents (phenothiazines (thioridazine, chlorpromazine, mesoridazine), butyrophenones (droperidol, haloperidol), and loperamide)).
6. Previously confirmed long QT syndrome.
7. Uncontrolled blood pressure at the time of screening, defined as a systolic pressure > 140 mmHg or <90 mmHg, or a diastolic pressure < 90 mmHg or > 50 mmHg.
8. Heart rate < 50 bpm or > 90 bpm at the time of screening.
9. Active or untreated malignancies within 5 years before the screening, or in clinical remission of malignancies (with the exception of patients with no recurrence after surgery for basal cell carcinoma and squamous cell carcinoma, cervical carcinoma *in situ,* papillary thyroid carcinoma).
10. A history of acute or chronic pancreatitis, or serum lipase/amylase 2 times greater than the upper limit of normal (ULN) at the time of screening, or fasting triglyceride > 5.65 mmol/L (500 mg/dL). If the patient is receiving a lipid-regulating treatment, the dose of the treatment must be stable for 30 days before the screening.
11. At the time of screening, liver diseases with clinical symptoms, acute or chronic hepatitis, or transaminase (ALT and AST) and alkaline phosphatase (ALP) > 2 × ULN, and total bilirubin > ULN.
12. Calcitonin ≥ 15 ng/L at the time of screening.
13. Estimated glomerular filtration rate (eGFR) < 60 mL/min/1.73 m² at the time of screening using the modified MDRD equation: eGFR = 175 × [(serum creatinine (µmol/L)/88.4)] - 1.234 × [age (years)] - 0.179 × 0.79 (female) or × 1 (male).
14. Ineligibility due to previous or current mental disorders at the time of screening as determined by the investigator.
15. Confirmed gastroparesis or any form of bariatric surgery, or abnormal gastric emptying with clinical significance as determined by the investigator.
16. A history of known regular drug abuse.
17. HIV infection, and/or HIV antibody positivity or syphilis antibody positivity at the time of screening.
18. A history of hepatitis B, and/or hepatitis B surface antigen positivity or hepatitis C virus (HCV) antibody positivity at the time of screening.
19. Previous Gilbert syndrome.
20. INR > ULN at the time of screening.
21. A history of medullary thyroid cancer, a history of MEN (multiple endocrine neoplasia) 2A or 2B syndrome, or a related family history.
22. Previously confirmed autonomic neuropathy, indicated by: urinary retention, resting tachycardia, orthostatic hypotension, and diabetic diarrhea.
23. Significant body weight change within 3 months before the screening (> 5%).
24. Blood donation of > 400 mL or excessive blood loss or bone marrow transplantation within 3 months before the screening, or the presence of hemoglobinopathy, hemolytic anemia, sickle-cell anemia, or hemoglobin < 110 g/L (male) or < 100 g/L (female).
25. Clinically assessed and/or TSH abnormality-confirmed hyperthyroidism or hypothyroidism that may increase risks in the patient as determined by the investigator.
26. Use of hypoglycemic agents other than metformin within 2 months before the screening.
27. Use within 3 months before the screening of weight-loss drugs such as liraglutide, orlistat, sibutramine hydrochloride, phenylpropanolamine, mazindol, phentermine, lorcaserin hydrochloride, phentermine, phentermine/topiramate, amfepramone and naltrexone/amfepramone, or planned use during the study.
28. Long-term use of glucocorticoids within 1 year before the screening (> 2 weeks of cumulative or continuous use), or use of glucocorticoids within 4 weeks before the screening (with exceptions of topical, intraocular, intranasal and intraarticular administrations, and inhalation).
29. Use of central nervous system stimulants (e.g., methylphenidate hydrochloride) at the time of screening, with the exception of caffeine-containing beverages.
30. Known allergy to the study drugs or components.
31. Participation in clinical trials of any drug or medical device (defined as the start of random treatment) within 3 months before the screening.
32. Female subjects with child-bearing potential who are unwilling to inform their partners of their participation in this clinical study or unwilling to take effective contraceptive measures during the study, except for those who have been sterilized or are menopausal. or male subjects who are unwilling to inform their partners of their participation in this clinical study or unwilling to take effective contraceptive measures during the study.
33. Pregnant or lactating women, or subjects with planned pregnancy or breast-feeding during the study.
34. Any clinically significant laboratory test outliers that may interfere with the interpretation of the efficacy and safety data of the study as determined by the investigator.
35. Other factors that may affect the compliance of the subjects or efficacy or safety assessment as determined by the investigator, such as mental disorders.
36. Over 21 units of alcohol use for men or over 14 units for women per week on average, or unwillingness to cease drinking within 24 hours before dosing and throughout the study period (1 unit = 360 mL of beer, or 150 mL of wine, or 45 mL of distilled spirits).

### Example 2. Overall study design

### I. For obesity or overweight

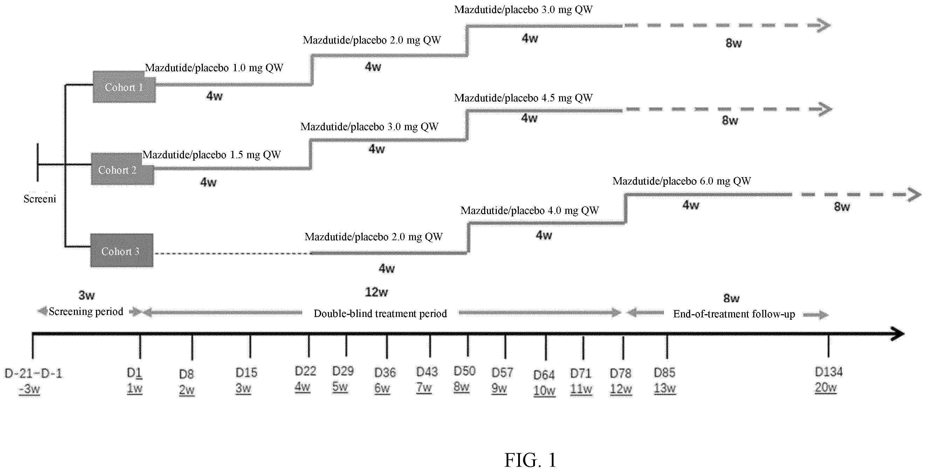

In this study, the enrollment of 36 patients with overweight or obesity having a weight change of less than 5% after at least 12 weeks of diet/exercise control was planned. Three cohorts were included in this double-blind study: cohort 1 (n = 12), cohort 2 (n = 12), and cohort 3 (n = 12). Subjects in each cohort were randomized in a 2:1 ratio into the mazdutide treatment group (n = 8) and the placebo group (n = 4). The subcutaneous dosing regimens for mazdutide or placebo in cohort 1, cohort 2 and cohort 3, are described below (as shown in FIG. 1):
***Cohort 1:*** Subjects were administered with a starting dose of 1.0 mg once weekly for 4 weeks. If the treatment was well tolerated*, the dose was adjusted to 2.0 mg, and was administered once weekly for 4 weeks. If the treatment was well tolerated*, the dose was adjusted to 3.0 mg, and was administered once weekly for 4 weeks. (ascending at a rate of 1 mg/4 weeks to the target dose).
***Cohort 2:*** Subjects were administered with a starting dose of 1.5 mg once weekly for 4 weeks. If the treatment was well tolerated*, the dose was adjusted to 3.0 mg, and was administered once weekly for 4 weeks. If the treatment was well tolerated*, the dose was adjusted to 4.5 mg, and was administered once weekly for 4 weeks (ascending at a rate of 1.5 mg/4 weeks to the target dose).

If intolerance was observed in subjects in cohort 2 at 3.0 mg or 4.5 mg, the dose was adjusted according to the standard in Table 3.

**Table 3. Reference for dose escalation and adjustment**

| | | | | | | | | | | | | | | | | | |
|---|---|---|---|---|---|---|---|---|---|---|---|---|---|---|---|---|---|
| **Week** | | **1** | **2** | **3** | **4** | **5** | **6** | **7** | **8** | **9** | **10** | **11** | **12** | | | | |
| Cohort 1 (N=12) | mazdutide/Placebo | 1.0mgQW | | | | 2.0mgQW | | | | 3.0mgQW | | | | | | | |
| **Week** | | **1** | **2** | **3** | **4** | **5** | **6** | **7** | **8** | **9** | **10** | **11** | **12** | | | | |
| Cohort 2 (N=12) | mazdutide/Placebo | 1.5mgQW | | | | 3mgQW | | | | 4.5mgQW | | | | | | | |
| **Week** | | **1** | **2** | **3** | **4** | **5** | **6** | **7** | **8** | **9** | **10** | **11** | **12** | | | | |
| Cohort 2 (backup 1)* | mazdutide/Placebo | 1.5mgQW | | | | 3mg | 0mg | 2.25mg | 2.25mg | 3mgQW | | | | | | | |
| **Week** | | **1** | **2** | **3** | **4** | **5** | **6** | **7** | **8** | **9** | **10** | **11** | **12** | | | | |
| Cohort 2 (backup 2)^{#} | mazdutide/Placebo | 1.5mgQW | | | | 3mgQW | | | | 4.5mg | 0mg | 3.75mg | 3.75mg | | | | |
| **Week** | | **-4** | **-3** | **-2** | **-1** | **1** | **2** | **3** | **4** | **5** | **6** | **7** | **8** | **9** | **10** | **11** | **12** |
| Cohort 3 (N=12)^{&} | mazdutide/Placebo | | | | | 2.0mgQW | | | | 4.0mgQW | | | | 6.0mg | | | |

| | | | | | | | | | | | | | | | | | |
|---|---|---|---|---|---|---|---|---|---|---|---|---|---|---|---|---|---|
| Notes: * If 3 mg was not tolerated in the subjects in cohort 2, the treatment in cohort 2 (backup 1) should be discontinued for 1 week and resumed at the dose of 2.25 mg, and the subsequent dose escalation was conducted in cohort 2 (backup 1); * If 4.5 mg was not tolerated in the subjects in cohort 2, the treatment in cohort 2 (backup 2) should be discontinued for 1 week and resumed at the dose of 3.75 mg until the end of study; & Treatment in cohort 3 was started when the 4-week treatment at 1.5 mg was tolerated in the subjects in cohort 2; if 1.5 mg was not tolerated in cohort 2, 2.0 mg and higher doses were not investigated in cohort 3. ***Cohort 3:*** Treatment in cohort 3 was started when the 4-week treatment at 1.5 mg was completed and well tolerated in the subjects in cohort 2; if 1.5 mg was not tolerated in cohort 2, 2.0 mg and higher doses were not investigated in cohort 3. In this cohort, subjects were administered with a starting dose of 2.0 mg once weekly for 4 weeks. If the treatment was well tolerated*, the dose was adjusted to 4.0 mg, and was administered once weekly for 4 weeks. If the treatment was well tolerated*, the dose was adjusted to 6.0 mg, and was administered once weekly for 4 weeks. (ascending at a rate of 2 mg/4 weeks to the target dose). | | | | | | | | | | | | | | | | | |

### II. For diabetes

In this study, the enrollment of 42 patients with type 2 diabetes with uncontrolled glycosylated hemoglobin despite at least 2 months of lifestyle intervention or treatment with a stable dose of metformin (≥1000 mg/day or the maximum tolerated dose). Three cohorts were included in this study: cohort 1 (n = 14), cohort 2 (n = 14), and cohort 3 (n = 14). Subjects in each cohort were randomized in an 8:4:2 ratio into the mazdutide treatment group (n = 8), the placebo group (n = 4), and the dulaglutide 1.5 mg treatment group (n = 2). In cohorts 1, 2, and 3, the active control drug, dulaglutide, was administered at 1.5 mg QW for 12 weeks, and the dosing regimens for mazdutide and placebo are described below:
***Cohort 1:*** Subjects were administered with a starting dose of 1.0 mg once weekly for 4 weeks. If the treatment was well tolerated*, the dose was adjusted to 2.0 mg, and was administered once weekly for 4 weeks. If the treatment was well tolerated*, the dose was adjusted to 3.0 mg, and was administered once weekly for 4 weeks. (ascending at a rate of 1 mg/4 weeks to the target dose).
***Cohort 2:*** Subjects were administered with a starting dose of 1.5 mg once weekly for 4 weeks. If the treatment was well tolerated*, the dose was adjusted to 3.0 mg, and was administered once weekly for 4 weeks. If 3.0 mg was not well tolerated but still met the tolerance criteria, the next dose was investigated in cohort 2 (backup 1); if the dose was well tolerated, the study proceeded to 4.5 mg. The treatment was given once weekly for 4 weeks, if 4.5 mg was not well tolerated but still met the tolerance criteria, the next dose was investigated in cohort 2 (backup 2). (ascending at a rate of 1.5 mg/4 weeks to the target dose).
***Cohort 2*** (backup 1): If 3.0 mg was not tolerated, the treatment should be discontinued for 1 week and resumed at the dose of 2.25 mg for 2 weeks; when further observation suggested a good tolerance, the treatment was returned to 3.0 mg for 4 weeks.
***Cohort 2*** (backup 2): If 4.5 mg was not tolerated, the treatment should be discontinued for 1 week and resumed at the dose of 3.75 mg for 2 weeks until the end of study.
***Cohort 3:*** Treatment in cohort 3 was started when the 4-week treatment at 1.5 mg was completed in the subjects in cohort 2; if 1.5 mg was not tolerated in cohort 2, 2.0 mg and higher doses were not investigated in cohort 3. In this cohort, subjects were administered with a starting dose of 2.0 mg once weekly for 4 weeks. If the treatment was well tolerated, the dose was adjusted to 4.0 mg, and was administered once weekly for 4 weeks. If the treatment was well tolerated, the dose was adjusted to 6.0 mg, and was administered once weekly for 4 weeks. (ascending at a rate of 2 mg/4 weeks to the target dose).

### Example 3. Safety assessments

For patients with obesity or overweight: By March 15, 2021, 36 subjects were enrolled and 36 were included in the analysis. The investigational product showed good overall safety and tolerance in the subjects throughout the study, with no SAEs, no dose escalation termination-related AEs, no hypoglycemia events, no severe adverse events, no acute pancreatitis, no discontinuation or withdrawal due to AEs, and no injection site reactions does not occur. Only 2 patients in cohort 1 had mild urticaria. Gastrointestinal adverse events were the most common (15/24 cases, 62.5%), among which anorexia (29.2%), diarrhea (25%), and nausea (16.7%) demonstrated the highest rates of occurrence. The gastrointestinal events occurred at a higher rate in the treatment group than in the placebo group, which complies with the mechanism of action of the drug. In addition, the mazdutide treatment group had an increase in average heart rate over the placebo group, but no severe adverse events associated with heart disease were found.

For patients with diabetes: By April 26, 2021, 42 subjects were enrolled and 42 were included in the analysis. The investigational product showed good overall safety and tolerance in the subjects throughout the study, with no dose escalation termination-related AEs and no acute pancreatitis, severe hypoglycemia, allergic reaction or injection site reaction.

### Example 4. Effect of mazdutide on reducing uric acid based on clinical safety study

1. For subjects with obesity: By March 15, 2021, 36 subjects were enrolled and 36 were included in the analysis. Table 4 illustrates the pre-dose uric acid levels of each group, i.e., baseline values.

**Table 4**

| **Baseline** | **Cohort 1** | **Cohort 2** | **Cohort 3** | **Placebo (N=12)** |
|---|---|---|---|---|
| | **1 mg-2 mg-3 (N=8)** | **mg 1.5 mg-3 mg-4.5 (N=8)** | **mg 2 mg-4 mg-6 mg (N=7)** | |
| **Uric acid (µmol/L)** | **375.4(144.20)** | **412.4(84.43)** | **416.5(107.79)** | **349.4(72.26)** |
| **Mean (SD)** | | | | |

FIG. 2 illustrates the change from baseline. As can be seen in FIG. 2, cohorts 1, 2, and 3 all had significant reductions in uric acid levels, which were significantly superior to those of the placebo group.
2. For subjects with diabetes: By April 26, 2021, 42 subjects were enrolled and 36 were included in the analysis. Table 5 illustrates the pre-dose uric acid levels of each group, i.e., baseline values.

**Table 5**

| **Baseline** | **Cohort 1 (N=7)** | **Cohort 2 (N=5)** | **Cohort 3 (N=8)** | **Placebo (N=10)** | **Dulaglutide (N=6)** |
|---|---|---|---|---|---|
| **Uric acid (µmol/L)** | **315.57 (77.214)** | **295.80 (117.393)** | **281.13 (79.316)** | **287.40 (53.800)** | **301.33 (58.909)** |
| **Mean (SD)** | | | | | |

Table 6 shows the mean change in the groups on day 85 post-dose as compared to the baseline. It can be seen from Table 6 and FIG. 3 that cohorts 1, 2, and 3 had significantly reduced uric acid levels than the placebo group and the dulaglutide control group, among which cohorts 1 and 3 had the most significant reductions.

**Table 6**

| **Baseline** | **Cohort 1 (N=7)** | **Cohort 2 (N=5)** | **Cohort 3 (N=8)** | **Placebo (N=10)** | **Dulaglutide (N=6)** |
|---|---|---|---|---|---|
| **Uric acid (µmol/L)** | **-38.14 (36.957)** | **0.52 (69.226)** | **-32.50 (40.981)** | **9.40 (48.021)** | **16.67 (30.533)** |
| **Mean of change (SD)** | | | | | |

## Claims

1. Use of a compound of formula (I) or a pharmaceutically acceptable salt thereof in preparing a medicament for reducing a uric acid level in a patient;

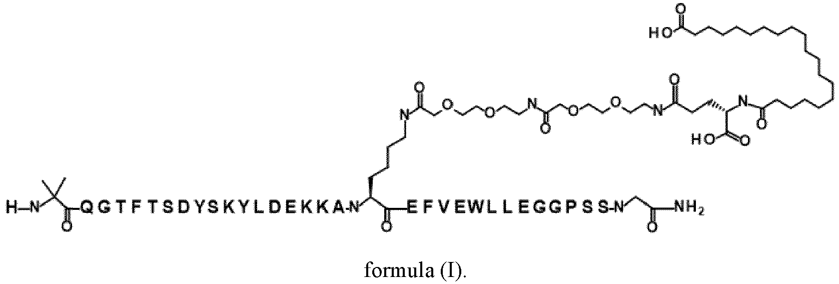

2. The use according to claim 1, wherein the compound or the pharmaceutically acceptable salt thereof is the sole active ingredient or one of the active ingredients in the medicament.

3. The use according to claim 1 or 2, wherein the medicament further comprises tris(hydroxymethyl)aminomethane and mannitol, and preferably further comprises sucrose or propylene glycol.

4. The use according to any one of claims 1-3, wherein the uric acid level is greater than 280 µmol/L, or/and the patient has gout, hyperuricemia, uremia, atherosclerosis, hypertension, fatty liver, diabetes, obesity, or overweight with complications; the uric acid level is a serum uric acid level in the patient before the reduction.

5. The use according to claim 4, wherein the uric acid level is greater than 420 µmol/L.

6. A method for reducing a uric acid level in a patient, comprising administering to the patient an effective amount of a compound of formula (I) or a pharmaceutically acceptable salt thereof;

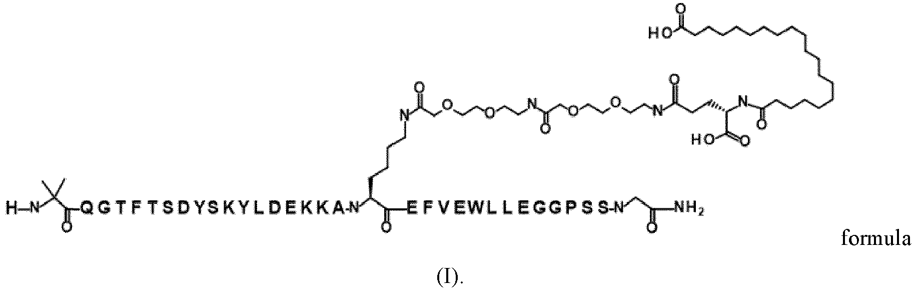

7. The method according to claim 6, wherein the compound or the pharmaceutically acceptable salt thereof is the sole active ingredient or one of the active ingredients in a medicament.

8. The method according to claim 6 or 7, wherein the medicament further comprises tris(hydroxymethyl)aminomethane and mannitol, and preferably further comprises sucrose or propylene glycol.

9. The method according to any one of claims 6-8, wherein the uric acid level is greater than 280 µmol/L, or/and the patient has gout, hyperuricemia, uremia, atherosclerosis, hypertension, fatty liver, diabetes, obesity, or overweight with complications; the uric acid level is a serum uric acid level in the patient before the reduction.

10. The method according to claim 9, wherein the uric acid level is greater than 420 µmol/L.

11. The method according to any one of claims 6-8, wherein the medicament is administered at a dose of 1.0-10 mg once weekly; preferably, the medicament is administered at a dose of about 1.0 mg, 1.5 mg, 2.0 mg, 2.5 mg, 3.0 mg, 4.0 mg, 4.5 mg, 5 mg, 6 mg, 7.5 mg, 9 mg, or 10 mg once weekly;
more preferably, the medicament is administered at at least one ascending dose about once weekly for at least about four weeks, and administered at at least one maintenance dose about once weekly for at least about four weeks after the ascending dose; wherein the ascending dose is selected from about 1.0 mg and about 2.0 mg; wherein the maintenance dose is selected from about 3.0 mg; or
the medicament is administered at at least one ascending dose about once weekly for at least about four weeks, and administered at at least one maintenance dose about once weekly for at least about four weeks after the ascending dose; wherein the ascending dose is selected from about 1.5 mg and about 3.0 mg; wherein the maintenance dose is selected from about 4.5 mg; or
the medicament is administered at at least one ascending dose about once weekly for at least about four weeks, and administered at at least one maintenance dose about once weekly for at least about four weeks after the ascending dose; wherein the ascending dose is selected from about 2.0 mg and about 4.0 mg; wherein the maintenance dose is selected from about 6.0 mg.

12. A pharmaceutical composition for reducing a uric acid level in a patient, comprising a compound of formula (I) or a pharmaceutically acceptable salt thereof, and at least one pharmaceutically acceptable carrier;

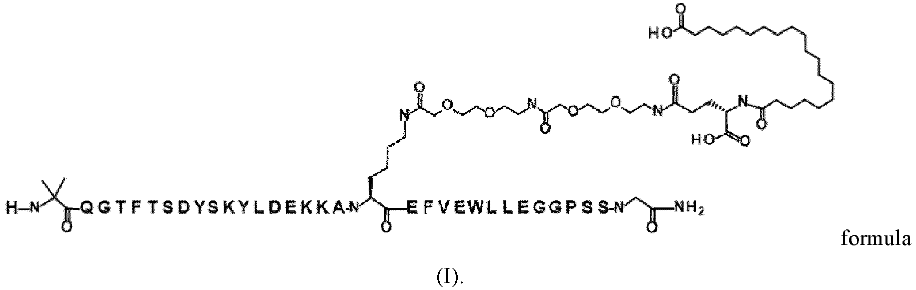

13. The pharmaceutical composition according to claim 12, wherein the compound or the pharmaceutically acceptable salt thereof is the sole active ingredient or one of the active ingredients in a medicament.

14. The pharmaceutical composition according to claim 12 or 13, wherein the medicament further comprises tris(hydroxymethyl)aminomethane and mannitol, and preferably further comprises sucrose or propylene glycol.

15. The pharmaceutical composition according to any one of claims 12-14, wherein the uric acid level is greater than 280 µmol/L, or/and the patient has gout, hyperuricemia, uremia, atherosclerosis, hypertension, fatty liver, diabetes, obesity, or overweight with complications; the uric acid level is a serum uric acid level in the patient before the reduction.

16. The pharmaceutical composition according to claim 15, wherein the uric acid level is greater than 420 µmol/L.

17. The pharmaceutical composition according to any one of claims 12-14, wherein the medicament is administered at a dose of 1.0-10 mg once weekly; preferably, the medicament is administered at a dose of about 1.0 mg, 1.5 mg, 2.0 mg, 2.5 mg, 3.0 mg, 4.0 mg, 4.5 mg, 5 mg, 6 mg, 7.5 mg, 9 mg, or 10 mg once weekly;
more preferably, the medicament is administered at at least one ascending dose about once weekly for at least about four weeks, and administered at at least one maintenance dose about once weekly for at least about four weeks after the ascending dose; wherein the ascending dose is selected from about 1.0 mg and about 2.0 mg; wherein the maintenance dose is selected from about 3.0 mg; or
the medicament is administered at at least one ascending dose about once weekly for at least about four weeks, and administered at at least one maintenance dose about once weekly for at least about four weeks after the ascending dose; wherein the ascending dose is selected from about 1.5 mg and about 3.0 mg; wherein the maintenance dose is selected from about 4.5 mg; or
the medicament is administered at at least one ascending dose about once weekly for at least about four weeks, and administered at at least one maintenance dose about once weekly for at least about four weeks after the ascending dose; wherein the ascending dose is selected from about 2.0 mg and about 4.0 mg; wherein the maintenance dose is selected from about 6.0 mg.
